# EUROPEAN PATENT APPLICATION

(11) **EP 1 902 744 A1**
(43) Date of publication of application: **26.03.2008**
(21) Application number: 07075829.7
(22) Date of filing: 21.09.2007
(51) Int. Cl.: A61M 16/08, A61M 16/10, A61M 16/20

(54) **Breathing circuit comprising a heater wire and a pressure relief valve**

(30) Priority: 21.09.2006 US 524328
(71) Applicant: Teleflex Medical Incorporated, Durham, NC 27709 (US)
(72) Inventor: Roth, Gary, Wake Forest,NC 27587 (US); Fitzwater, Dennis, Coronado, CA 92118-2835 (US); Burk, Mark, Murrieta, CA 92563 (US)
(74) Representative: Kortekaas, Marcel C.J.A.

(57) **Abstract**

A respiratory breathing circuit (12) prevents heat loss and condensation in a flow of humidified air through a conduit from a humidification system (10) to a patient interface (36), such as a nasal cannula. The circuit directly impedes heat loss or insulates the flow of a humidified gas therein, and provides a heating source (38) inside the circuit along the length of the circuit to prevent net heat loss from the circuit tubing by providing thermal energy and/or insulation to impede convective, conductive, or radiative heat loss by the gas as it flows through the system. A temperature, humidity, or other flow quality measuring probe (32) is disposed at the distal end of the circuit near the patient interface to actively measure a corresponding quality of the humidified gas flow to provide feedback to the system. A pressure relief valve (44) is provided on the circuit to prevent a pressure build-up and/or structural failure.

## Description

### FIELD OF THE INVENTION

The present invention relates generally to medical devices. More particularly, the present invention relates to a respiration circuit for providing a continuous high flow of heated and humidified and heated gases to a patient.

### BACKGROUND OF THE INVENTION

Respiratory therapy systems using mechanical ventilation for moving gas into a patient's lungs commonly incorporate a humidifier along the respiratory circuit in order to heat or humidify the respiratory gas directed to the patient. Examples of such humidifiers are disclosed in U.S. Pat. Nos. 4,110,419, 4,172,105, 4,195,044, 4,500,480 and 4,674,494. Ventilator circuits and other tubing apparatus are designed to direct breathing gas to the patient, with a ventilator or other gas source supplying the gas to be breathed under pressure or at other elevated flow rates at breathing rates and breath gas volumes prescribed to meet the patient's requirements.

Typically, the breathing gas is humidified by a humidifier located at or near the ventilator or gas source whereby the humidified gas must travel substantially the entire length of the circuit or tubing. The temperature of the gases within the humidifier, when delivered to the patient, is typically 37 degrees C, while room temperature is typically in the vicinity of 22 degrees C. The humidified gas becomes cooled along the tubing length resulting in condensation or "rainout" within the tubing which requires routine maintenance by the attending clinician. Should the clinician not be diligent in managing this rainout, a bolus of water can then be drawn into the airway of a patient, causing significant harm. Some respiratory circuits are provided with water traps or other means for removing condensate from the respirator tubing which would otherwise interfere with gas delivery. Alternatively, ventilator or respiratory circuits may be provided with heater wire extending along the interior of the tubing or embedded in or otherwise secured along the wall of the tubing. Examples of such heated ventilator or respiratory circuits are described in U.S. Pat. Nos. 4,682,010, 5,640,951 and 5,537,996. These systems can use various features to improve performance, including the use of temperature probes along the length of the tubing to provide feedback to the system and accordingly adjust the supply of warmed air.

However, no existing circuit for providing respiratory therapy to a patient uses adequate measures to ensure a continuous supply of high volume heated and humidified air, at flow rates of up to 40 liters per minute, so as to effectively and efficiently eliminate the rain-out problem, as well as to provide adequate safety measures, so as to ensure that the air delivered to the patient is at the proper temperature, pressure, and humidity level.

Accordingly, it is desirable to provide a method and apparatus for a respiratory breathing circuit that prevents heat loss and condensation in a flow of humidified air supplied through a conduit from a humidification system to a patient interface, such as a nasal cannula, and which provides adequate safety measures to ensure that the air delivered to the patient is at the proper therapeutic conditions.

### SUMMARY OF THE INVENTION

The foregoing needs are met, to a great extent, by the present invention, wherein in one aspect an apparatus is provided that in some embodiments provides a respiratory breathing circuit that prevents heat loss and condensation in a flow of humidified air supplied through a conduit from a humidification system to a patient inter-face, such as a nasal cannula. The present invention accomplishes this by providing a number of configurations in the breathing circuit that either: (a) directly impede heat loss or insulate the flow of a humidified gas therein, or (b) provide a heating source inside the circuit along the length of the circuit to indirectly prevent net heat loss from the circuit tubing by providing thermal energy that is lost to convective, conductive, or radiative heat loss by the gas as it flows through the system. In either case, the present invention utilizes a temperature and/or humidity, or other flow quality measuring probe disposed at the distal end of the circuit near the patient interface to actively measure the corresponding quality of the humidified gas flow so as to provide feedback and information to the system. An additional safety device in the form of a pressure relief valve may be included with the circuit architecture to prevent a pressure build-up and/or structural failure, as well as to alert the surroundings of such an event.

In accordance with one embodiment of the present invention, a respiratory breathing circuit is provided, having a tubular conduit having proximal and distal end portions. The distal end portion is configured to be directly proximate a patient interface for administering a respiratory breathing flow to a patient. The conduit defines a flow lumen having a heater wire partially disposed in the lumen. A temperature or flow quality monitoring port is disposed at the distal end portion of the tubular conduit, in fluid communication with the flow lumen. A pressure relief valve is disposed on the at least one tubular conduit in fluid communication with the flow lumen. A temperature probe can be coupled to the flow quality monitoring port, and/or a moisture measuring probe can be coupled to the flow quality monitoring port. The circuit can also have a patient interface coupled to the distal end portion of the tubular conduit. In one embodiment, the patient interface includes a cannula loop having proximal and distal halves, with the proximal half of the loop having passive insulation surrounding the loop. A junction is disposed at the proximal end portion of the tubular conduit, having a first port for receiving the heater wire, and a second port for receiving a fluid flow int0 the lumen. The tubular conduit further includes a strain relief means incorporated int0 a portion of the tubular conduit immediately distal to the junction, such that the strain relief means reinforces the structural integrity of the tubular conduit. The strain relief means can be a corrugation means, or can include a coiled element.

In yet another aspect of the present invention, a respiratory breathing circuit is provided, having at least one tubular conduit having proximal and distal end portions. The distal end portion is configured to be directly proximate a patient interface for administering a respiratory breathing flow to a patient. The tubular circuit defines at least one lumen for a fluid flow. A means is disposed in, or incorporated or integrated as a part of, the tubular conduit for impeding heat loss in the fluid flow. A flow quality monitoring port is disposed at the distal end portion of the tubular conduit, in fluid communication with the at least one lumen. A pressure relief valve is disposed on the tubular conduit in fluid communication with the lumen.

In still another aspect of the present invention, a method of providing a respiratory breathing gas flow to a patient is disclosed. A flow of fluid is supplied through at least one tubular conduit having proximal and distal end portions. The tubular conduit defines at least one lumen for a fluid flow. The loss of heat by the flow of fluid along a length of the tubular conduit is actively impeded. And, a flow quality of the flow of fluid is measured through a flow quality monitoring port disposed at the distal end portion of the tubular conduit, in fluid communication with the lumen. The flow of fluid is exposed to a pressure relief valve disposed on a least one tubular conduit, the valve being calibrated to open and release the flow of fluid from the lumen at a predetermined overpressure condition.

There has thus been outlined, rather broadly, certain embodiments of the invention in order that the detailed description thereof herein may be better understood, and in order that the present contribution to the art may be better appreciated. There are, of course, additional embodiments of the invention that will be described below and which will form the subject matter of the claims appended hereto.

In this respect, before explaining at least one embodiment of the invention in detail, it is to be understood that the invention is not limited in its application to the details of construction and to the arrangements of the components set forth in the following description or illustrated in the drawings. The invention is capable of embodiments in addition to those described and of being practiced and carried out in various ways. Also, it is to be understood that the phraseology and terminology employed herein, as well as the abstract, are for the purpose of description and should not be regarded as limiting.

As such, those skilled in the art will appreciate that the conception upon which this disclosure is based may readily be utilized as a basis for the designing of other structures, methods and systems for carrying out the several purposes of the present invention. It is important, therefore, that the claims be regarded as including such equivalent constructions insofar as they do not depart from the spirit and scope of the present invention.

### BRIEF DESCRIPTION OF THE DRAWINGS

FIG. 1 is a schematic view illustrating a high flow respiratory breathing system and circuit according to one embodiment of the invention.

FIG. 2 is a plan view of the distal end portion of the respiratory breathing system and circuit of FIG.1, as well as a nasal cannula patient interface.

FIG. 3 is a plan view showing the proximal end portion of the circuit, including a junction.

FIG. 4 is a view taken along the line A--A in FIG. 3, showing a close-up of the junction at the proximal end portion of the circuit.

### DETAILED DESCRIPTION

The invention will now be described with reference to the drawing figures, in which like reference numerals refer to like parts throughout. An embodiment in accordance with the present invention provides a respiratory breathing circuit that prevents heat loss and condensation in a flow of humidified air supplied through a conduit from a humidification system to a patient interface, such as a nasal cannula. The circuit is part of an overall gas humidification system that supplies a high flow of respiratory breathing air to a patient through a patient interface. The present invention directly impedes heat loss and/or insulates the flow of a humidified gas through the circuit, and in one embodiment provides a heating source inside the circuit along the length of the circuit to prevent net heat loss from the circuit tubing. A temperature, humidity, both or other flow quality measuring probe can be disposed at the distal end of the circuit near the patient interface to actively measure the corresponding quality of the humidified gas flow so as to provide feedback and information to the system. An additional safety device in the form of a pressure relief valve is provided on the circuit architecture to prevent a pressure build-up and/or structural failure, as well as to alert the surroundings of such an event.

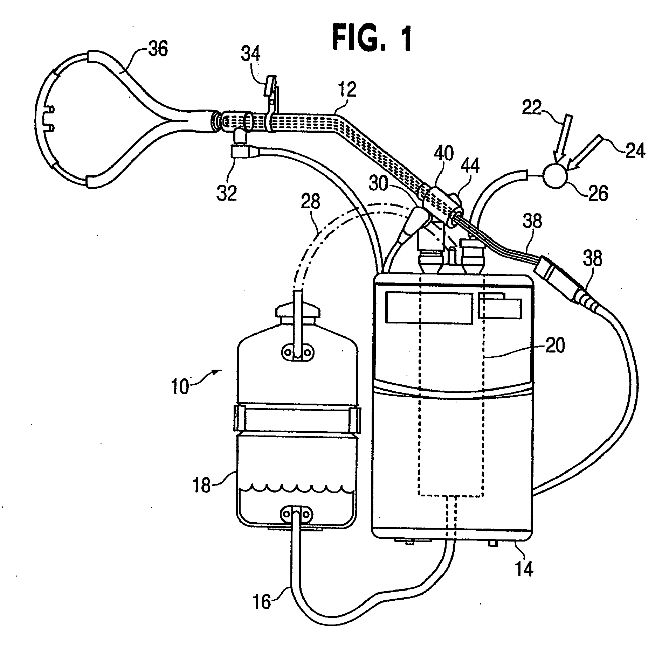

An embodiment of the present inventive apparatus is illustrated in FIG. 1. FIG. 1 is a schematic view illustrating a high flow respiratory breathing system and circuit according to one embodiment of the invention. The system 10 includes a respiratory breathing circuit 12, a heated humidifier 14 coupled via a water supply hose 16 to a water supply 18. The humidifier 14 includes a column 20 for adding moisture to a supply of air 22 or pure oxygen 24 through a mixing device 26. It is well understood that respiratory therapy utilizes a mixture of air or pure oxygen, but the principles of the present invention can be applied to the supply of any gas or mixture of gases and water. A water return 28 is also included between the humidifier column 14 and the water supply 18, thereby forming a closed system for adding moisture to the device.

The system 10 further includes at least two flow quality monitoring ports where a flow quality monitor or probe can be added. A first proximal probe 30 is provided at the proximal end portion of the circuit 12. In accordance with conventional practice, as used herein, the term "proximal" or "proximal end" shall refer to the specified end of a device or its component which is closer to the medical personnel handling or manipulating the device as it is intended to be used, and the term "distal" or "distal end" shall refer to the specified end of a device or its component which is closer to the patient. Another distal probe 32 can be provided at the distal end or distal end portion of the circuit 12. Either probe 30 or probe 32 can be a temperature measuring device or a moisture or relative humidity measuring device. A clip 34 is also provided at the proximal end portion of the circuit 12, which can be used to secure and attach a cable or wire, such as that connecting the distal end probe 32.

The "circuit" 12, as defined herein, shall be any arrangement of one or more tubular conduits connecting the supply of humidified gas, which can extend from the column all the way to a patient interface 36, where respiratory breathing is directly aided or enabled by the supply of the gases. The patient interface 36 can be any device which the patient wears or touches, which is directly proximate the patients mouth, nose, or throat. In the embodiment shown in FIG. 1, the patient interface is a nasal cannula having a loop shape. The patient interface can also be a face-mask, of rebreathing or non-rebreathing type, or any other device that directly couples a patient's respiratory system to a supply of air or gases for respiration.

By measuring the quality of the supplied gases to the patient at the distal end of the circuit 12, proximate the probe 32, the temperature or relative humidity of the supplied gases can be directly measured right before the gases are administered to the patient. This more effectively prevents any rain-out from occurring, since an undesirable temperature or moisture condition can be immediately measured at its more critical point, that is, just before the patient breathes the gases supplied.

The present invention is also supplied with a heating element. In the embodiment of FIG. 1, the heating element is a resistance heating wire 38 that is disposed through a junction 40 at the proximal end portion of the circuit 12. The heating wire 38 can be any arrangement of wires, such as a woven braid, multi-strand, helical coil, mesh, or other arrangement that can fit in the interior lumen of the circuit 12. The embodiment shown in FIG. 1 has a single wire in the form of a loop that enters and exits the lumen of the circuit 12 through the junction 40. The heat generated by the wire provides thermal energy to the flow inside the circuit 12 such that any convective, conductive or radiative heat lost by the flow in the lumen as it travels in the circuit 12 is offset by the thermal energy supplied by the wire 38.

This is not the only way of preventing or impeding heat loss in the flow in the circuit 12. An alternative embodiment of the present invention can include a multi-lumen tube for circuit 12, having an inner lumen which carries the flow of gas to the patient, and one or more outer lumens that radially enclose or surround the inner flow lumen. A fluid such as heated water can be supplied through the one or more outer lumens, thereby insulating the flow of gas in the inner lumen, to thus prevent or impede the loss of heat from the gas flow in the circuit 12. Any prevention of heat loss will accordingly prevent condensation in the system, so as to prevent rain-out from occurring. A number of lumen configurations is possible, as is well known in the art, including outer lumens that completely surround the inner lumen, or individual lumens that supply fluid to and from the distal end portion of the circuit 12.

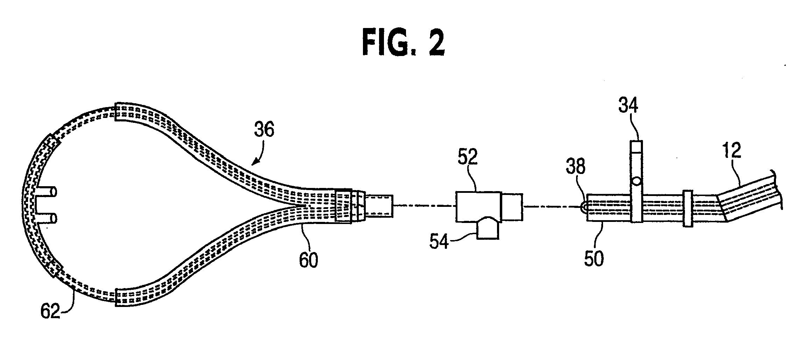

FIG. 2 is a plan view of the distal end portion of the respiratory breathing system 10 and circuit 12 of FIG. 1, including a nasal cannula patient interface 36. As can be seen in FIG. 2, the heater wire 38 can extend all the way along the circuit 12 to its distal end 50, where a junction or port 52 is disposed or coupled, The distal end 52 is configured to be directly proximate a patient interface 36 for administering a respiratory breathing flow to a patient. A flow quality monitoring port 54 is disposed at the distal end portion 50 of the circuit 12, which is in fluid communication with the flow lumen inside the circuit 12. any flow quality measuring device or probe can be disposed in the port 54, such as a temperature, pressure, or relative humidity or moisture measuring probe.

The patient interface 36 can be a loop shaped nasal cannula, which can include two halves, a proximal half 60 and a distal half 62. Passive insulation can be added to the proximal half 60 to prevent or impede heat loss or condensation in the flow lumen inside the cannula loop. This insulation is therefore a "passive" means, whereas the heater wire 38 or alternative multi lumen arrangements discussed above for the circuit 12 are active means of impeding heat loss in the fluid flow in the circuit 12.

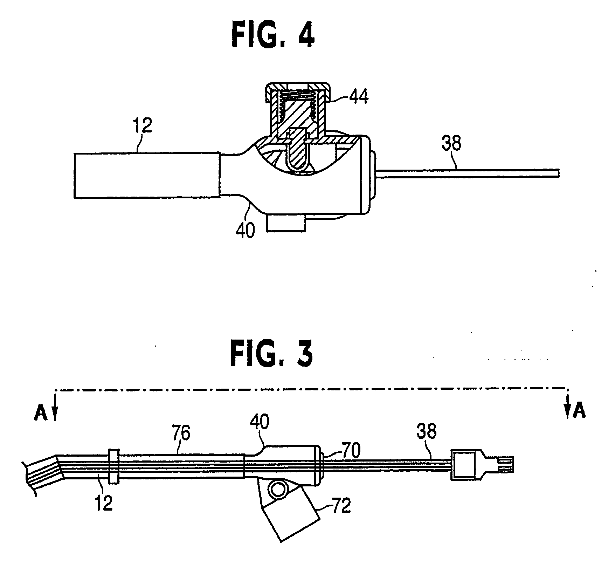

The circuit 12 can also include a pressure relief valve 44 at its proximal end, such as part of the junction 40, although the pressure relief valve 44 can be at any position along the circuit 12. HG. 3 is a plan view showing the proximal end portion of the circuit 12, including a junction 40. FIG. 4 is a view taken along the line A--A in HG. 3, showing a close-up of the junction 40 at the proximal end portion of the circuit 12. As can be seen in FIG. 3, the junction port 40 is disposed at the proximal end portion of the tubular conduit of the circuit 12, and includes a first port 70 for receiving the heater wire 38, and a second port 72 for receiving a fluid flow into the lumen. The first and second ports 70 and 72, preferably, form a non-perpendicular positive angle relative to one another in the plane shown in FIG. 3, so as to prevent kinking or tube occlusion from forming when heat is applied from the wire to the gas flow. A strain relief means 76 can be incorporated into a portion of the tubular conduit of the circuit 12 immediately distal to the junction 40, wherein the strain relief means 12 reinforces the structural integrity of the tubular conduit. The strain relief means can include a corrugation element or a coiled element. This also prevents kinking and occlusion from occurring.

Turning now to FIG. 4, a pressure relief valve 44 is shown, which is in fluid communication with the gas flow lumen in the circuit 12. If an overpressure condition develops the valve 44 can release gas or fluid from the conduit as necessary, or can be fitted with an audible element that alerts the surroundings of such an event. One example of an over-pressure condition would be when the gas pressure reaches 40 cm/H₂O for a neonatal, 1.8 psig for an adult, which can be damaging to the lungs and respiratory system of the patient, as well as the system apparatus.

As disclosed herein, the present invention efficiently and effectively supplies air to a patient for respiratory breathing that is at the correct temperature, humidity, and pressure conditions. The device can deliver up to 40 liters per minute, which is less for pediatric and neonatal patients. The system 10 can deliver to an adult patient a range of 5 to 40 liters per minute, a pediatric patent a range of 5 to 20 liters per minute, and a neonatal patient a range of 1 to 8 liters per minute. Patients will thus benefit from a high flow rate of heated, humidified oxygen, which can be used to alleviate hypoxemia, dyspnea, or other respiratory conditions in a hospital or home care setting.

The many features and advantages of the invention are apparent from the detailed specification, and thus, it is intended by the appended claims to cover all such features and advantages of the invention which fall within the true spirit and scope of the invention. Further, since numerous modifications and variations will readily occur to those skilled in the art, it is not desired to limit the invention to the exact construction and operation illustrated and described, and accordingly, all suitable modifications and equivalents may be resorted to, falling within the scope of the invention.

## Claims

1. A respiratory breathing circuit, comprising:
at least one tubular conduit having proximal and distal end portions and defining a flow lumen having a heater wire partially disposed in the lumen, the distal end portion being configured to be directly proximate a patient interface for administering a respiratory breathing flow to a patient,
a flow quality monitoring port disposed at the distal end portion of the at least one tubular conduit, in fluid communication with the flow lumen, and
a pressure relief valve disposed on the at least one tubular conduit in fluid communication with the flow lumen.

2. The circuit of claim 1, further comprising:
a temperature probe coupled to the flow quality monitoring port.

3. The circuit of claim 1 or 2, further comprising:
a moisture measuring probe coupled to the flow quality monitoring port.

4. The circuit of claim 1, 2 or 3 further comprising:
a patient interface coupled to the distal end portion of the tubular conduit, the patient interface having a cannula loop having proximal and distal halves, the proximal half of the loop having passive insulation surrounding the loop.

5. The circuit according to any of claims 1-4, further comprising:
a junction port disposed at the proximal end portion of the tubular conduit, having a first port for receiving the heater wire, and a second port for receiving a fluid flow into the lumen.

6. The circuit of claims 5, wherein the first and second ports form a non-perpendicular positive angle relative to one another in at least one plane.

7. The circuit of claim 5 or 6, wherein the tubular conduit comprises a strain relief means incorporated into a portion of the tubular conduit immediately distal to the junction, the strain relief means reinforcing the structural integrity of the tubular conduit.

8. The circuit of claim 7, wherein the strain relief means includes a corrugation means.

9. The circuit of claim 7, wherein the strain relief means includes a coiled element.

10. The circuit according to any of claims 5-9, wherein the pressure relief valve is incorporated on the junction.

11. A respiratory breathing circuit, comprising:
at least one tubular conduit having proximal and distal end portions and defining at least one lumen for a fluid flow, the distal end portion being configured to be directly proximate a patient interface for administering a respiratory breathing flow to a patient,
a means for impeding heat loss in the fluid flow in the at least one tubular conduit,
a flow quality monitoring port disposed at the distal end portion of the at least one tubular conduit, in fluid communication with the at least one lumen, and
a pressure relief valve disposed on the tubular conduit in fluid communication with the at least one lumen.

12. The circuit of claim 11, further comprising:
a temperature probe coupled to the flow quality monitoring port.

13. The circuit of claim 11 or 12, further comprising:
a moisture measuring probe coupled to the flow quality monitoring port.

14. The circuit of claim 11, 12 or 13 further comprising:
a junction disposed at the proximal end portion of the tubular conduit, having a first port for receiving the fluid flow into the at least one lumen from a source of humidified gas,
wherein the pressure relief valve is disposed on the junction.

15. The circuit of claim 14,
wherein a strain relief means is incorporated int0 a portion of the tubular conduit immediately distal to the junction, the strain relief means reinforcing the structural integrity of the tubular conduit.

16. A method of providing a respiratory breathing gas flow to a patient, comprising:
supplying a flow of fluid through at least one tubular conduit having proximal and distal end portions and defining at least one lumen for a fluid flow,
actively impeding the loss of heat by the flow of fluid along a length of the tubular conduit,
measuring at least one flow quality of the flow of fluid through a flow quality monitoring port disposed at the distal end portion of the at least one tubular conduit, in fluid communication with the at least one lumen, and
exposing the flow of fluid to a pressure relief valve disposed on the a least one tubular conduit, the valve being calibrated to open and release the flow of fluid from the lumen at a predetermined overpressure condition.

17. The method of claim 16, wherein the flow quality is temperature.

18. The method of claim 16 or 17, wherein the flow quality is relative humidity.

19. The method of claim 16, 17 or 18 further comprising the step of:
insulating a proximal half of a patient interface coupled to the distal end portion of the tubular conduit, the patient interface having a cannula loop.

20. The method according to any of claims 16-19, wherein the step of preventing the loss of heat by the flow of fluid along a length of the tubular conduit further comprises:
disposing a heater wire in the at least one lumen, and
heating the flow of fluid in the at least one lumen with the heater wire.

21. The method of claim 20, wherein the at least one tubular conduit further comprises:
a junction port disposed at the proximal end portion of the tubular conduit, having a first port for receiving the heater wire, and a second port for receiving a fluid flow into the lumen.

22. The method of claim 21, arranging the first and second parts such that the first and second parts form a non-perpendicular positive angle relative to one another in at least one plane.
